# EUROPEAN PATENT APPLICATION

(11) **EP 2 702 963 A1**
(43) Date of publication of application: **05.03.2014**
(21) Application number: 12006077.7
(22) Date of filing: 27.08.2012
(51) Int. Cl.: A61F 2/64

(54) **Powered prosthesis with serial and/or parallel compliance**

(71) Applicant: ETH Zürich, 8092 Zürich (CH)
(72) Inventor: Pfeifer, Serge, 8057 Zürich (CH); Riener, Robert, 8602 Wangen b. Dübendorf (CH); Keller, Urs, 8966 Oberwil-Lieli (CH); Vallery, Heike, 46282 Dorsten (DE); Lutz, Peter, 8455 Rüdlingen (CH)

(57) **Abstract**

A powered prosthesis with serial and/or parallel compliance, comprises a lower member (12) and an upper member (13), connected via a rotary joint (30), further comprising an actuator (20) connected with the lower member (12) and the upper member (13) to impart rotary motion to said lower member (12) and upper member (13) and a spring (25) connected with the actuator (20). The spring (25) is connected with actuator (20) in parallel and is attached between the lower member (12) and the upper member (13) at predetermined attachment points (23, 24) adapted that the force exerted by the parallel spring (25) supports the actuator in extension for a flexion angle between the lower member (12) and the upper member (13) between 20 and 90 degrees.

## Description

### TECHNICAL FIELD

The present invention relates to a powered prosthesis with serial and/or parallel compliance comprising a lower member and an upper member, connected via a rotary joint, further comprising an actuator connected with the lower member and the upper member to impart rotary motion to said lower member and upper member and at least one spring connected in series or in parallel with the actuator.

### PRIOR ART

Such a powered prosthesis is used to replace joints, to ensure the function of different joints e.g. the foot joint, the elbow joint or the knee joint. Relating to the replacement of the knee several problems arise. During natural gait, the stiffness of the human knee is modulated continuously, depending on the activity and the terrain. To enable natural locomotion, prosthetic devices should do the same. One way is to employ an actuator that can vary its stiffness intrinsically. Such an actuator is known from A. Jafari, N. Tsagarakis, and D. Caldwell in "AwAS-II: A new Actuator with Adjustable Stiffness based on the novel principle of adaptable pivot point and variable lever ratio," IEEE Int. Conf. Rob. Aut. 2011 (ICRA), pp. 4638-4643. Such actuators usually require a complex mechanical design and are difficult to incorporate in a transfemoral prosthesis, where the available space is limited and the required forces and torques are very high.

Another approach is to change the apparent stiffness of the device by appropriate control. This requires accurate force control. To achieve this, series-elastic actuators are employed. They also reduce shock loads, which are common loads in lower limb prostheses. Elastic elements can also be used to store energy. Series elastic actuators were first shown by G. A. Pratt, and M. M. Williamson, in "Series elastic actuators," IEEE/RSJ Int. Conf. on Intelligent Robots and Systems, pp. 399-406, 1995.

Behrens et al. have shown in S. M. Behrens, R. Unal, E. E. G. Hekman, R. Carloni, S. Stramigioli, and H. F. J. M. Koopman, "Design of a fully-passive transfemoral prosthesis prototype," Proc. Int. Conf. IEEE Eng. in Med. and Biol. Soc. 2011 (EMBC), pp. 591-594, 2011, a transfemoral prosthesis that generates knee and ankle torques similar to those during physiological level-ground walking, purely with springs, without an actuator. While this is extremely energy-efficient, it is specifically designed for level-ground walking, and activities that require positive kinetic energy, such as stair climbing, are not possible.

A powered ankle-foot prosthesis is known from S. Au, and H. Herr, "Powered ankle-foot prosthesis", IEEE Robotics & Automation Magazine, vol. 15, pp. 52 -59, 2008, using springs to optimize energy-efficiency during level-ground walking.

US 2009/0265018 A1 discloses a powered leg prosthesis and control methodologies for obtaining near normal gait from Goldfarb, providing an actuated knee and foot joint, using one linear actuator at each joint, wherein the actuator is an electric motor with a ballscrew transmission. The foot joint uses a spring in parallel to the actuator to improve the plantar flexion to obtain a higher torque at the end of the push-off action.

US 7314490 B3 discloses an actuated leg prosthesis for above-knee amputees by Bédard & Roy using a linear actuator. One embodiment provides a serial elasticity, wherein the spring is directly provided behind the actuator. This uses a lot of room, which is not acceptable for an artificial knee joint.

US 2010/0312363 A1 discloses a powered Artificial Knee with Agonist-Antagonist Actuation from Herr using two parallel actuators, one for flexion and one for extension, both having a serial elasticity, which allow to change the intrinsic stiffness of the joint without changing the torque.

Herr as an inventor of the MIT has published a further document with US 2011/0257764 A1 for a powered ankle-foot prosthesis comprising a serial and a parallel elasticity. The spring provided in parallel acts unidirectional in plantar flexion for a dorsiflexion angle larger than 0 degree, improving the actuator action when the foot is leaving the ground.

### SUMMARY OF THE INVENTION

Based on this prior art it is an object of the invention to provide a prosthesis allowing a better level-ground walking and readily allow stair climbing.

Mounting stairs with an artificial leg having an actuated knee joint needs quite an amount of positive energy which would lead to the use of heavy duty actuators which are impractical in an artificial leg intended to be similar to the leg to be replaced, due to weight and energy consumption constraints. It is a further object of the invention to provide an improved man-like prosthesis, which is better suited to help the impaired user of such an artificial leg to mount stairs.

The invention is based on the insight that a spring relieves the actuator during extension, where high torques are requested, and loads the actuator during flexion, where only low torques are needed. This allows the use of a smaller and lightweight actuator and/or an actuator using less energy.

This object is inter alia achieved with a prosthesis according to the invention that combines an actuator with series and/or parallel elastic elements. The invention is based on the insight that, in a physiological knee, extension is stronger than flexion and, therefore, bias torque is added, wherein the bias torque is nearly inexistent during usual free positions. Maximum bias torque is reached around 60 degree flexion.

The design according to the invention achieves a device capable of modulating its apparent stiffness in the range similar to the physiological knee as disclosed in Pfeifer, M. Hardegger, H. Vallery, R. List, M. Foresti, R. Riener, and E. J. Perreault, "Model-based estimation of active knee stiffness," IEEE Int. Conf. Rehabil. Robot. 2011 (ICORR), pp. 1-6, 2011, and to enable activities such as stair ascent and descent.

The series-elastic actuator is used to control the apparent stiffness of the device and to generate kinetic energy. The requirement for the physical stiffness or the series elasticity is derived from the stiffness capabilities of a physiological knee joint according to the paper from the inventors mentioned above, and the finding that the apparent stiffness a series-elastic actuator can display is bounded by the physical stiffness of the series-elastic element, as shown by Vallery, H.; Veneman, J.; van Asseldonk, E.; Ekkelenkamp, R.; Buss, M.; van Der Kooij, H. in "Compliant actuation of rehabilitation robots", IEEE Robotics & Automation Magazine, vol. 15 (3), pp 60-69, 2008.

Apart from the stiffness, the device must also be able to generate sufficient torque in order to approximate the capabilities of a physiological knee, and to at least reproduce the torques that are necessary for common activities such as stair ascent and descent.

The design can be optimized exploiting inter alia the finding that the highest torques occur over a limited range of knee angles. Furthermore, much higher torques are required in extension than in flexion. Then a parallel elastic element is added, which produces a torque in extension. This increases the total extension torque the device is able to output, and it reduces the flexion torque. The parallel spring is arranged such that little torque is produced near full extension of the knee joint, and also around 90 degrees flexion, such that the motor does not need to counteract the parallel spring when the amputee is standing or sitting.

The device according to the embodiment is able to reproduce the physiological knee torques during stair ascent and descent, and to modulate its apparent stiffness in a physiological range. As the power at the knee joint is mainly dissipative during level-ground walking and stair descent, a device according to the invention can also comprise a damper in parallel, to reduce energy consumption in those activities.

The design according to the invention results in a simple and lightweight artificial leg which is capable of modulating its apparent stiffness, and which is suitable for various activities.

It is a further advantage to use a linear actuator with a combination of two serial springs connected at the middle point using a rocker and attached serially to the linear actuator, which allows having a better use of space within the artificial shank and avoiding a mechanism which needs a stronger knee attachment as necessary with the parallel spring.

The actuator can for example be realized by a combination of rotary motor, ballscrew and a linear spring. The range of motion of the angle should be 120 degree and the weight of the entire structure should be as near as possible to the actual real leg, i.e. 4 to 4.5 kg. Modem passive prosthesis have a weight of around 1.2 kg, whereas active components bring additional weight.

The parallel spring allows use of actuators with reduced characteristics, since the maximum power to be produced by the actuator is reduced. When the knee flexion angle is higher than 90 degree the parallel spring acts in flexion, allowing the prosthesis to reach - while sitting - the mechanical end stop. When the knee flexion angle is less than 90 degree, the parallel spring acts in extension, with a maximum extension torque at around 60 degrees flexion angle. Near full extension, e.g. while standing, a small extension torque is provided, which relieves the actuator and avoids an easy buckling motion of the knee.

The use of the rocker allows the reduction of the actuator force to be transmitted on the springs. Thus, smaller and lighter springs can be used and the weight of the entire prosthesis can be reduced according to the invention. The separation of the positions of actuator and springs on both sides of the longitudinal shank axis provide a better weight distribution, similar to a physiological leg.

The upper attachment point is chosen to follow the physiological situation where the maximum possible knee torque changes with knee angle.

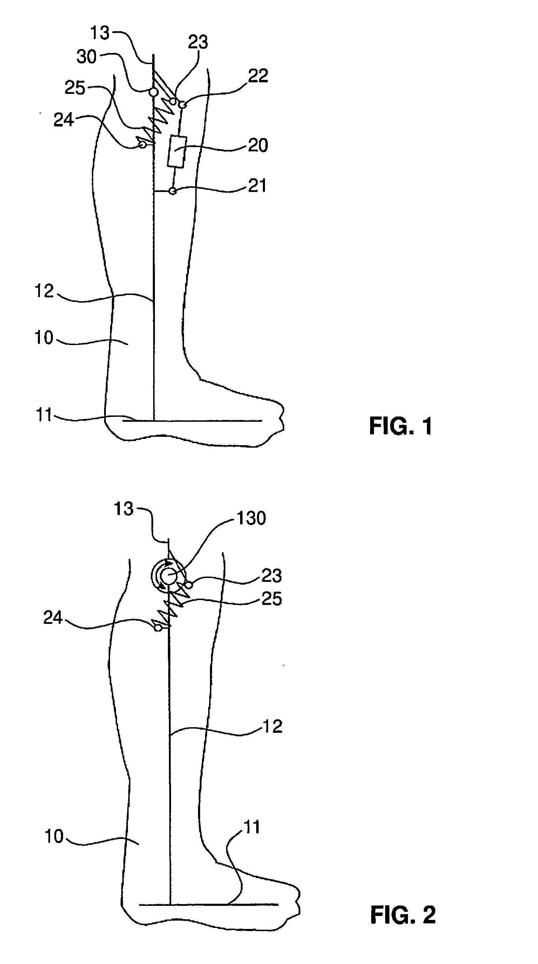

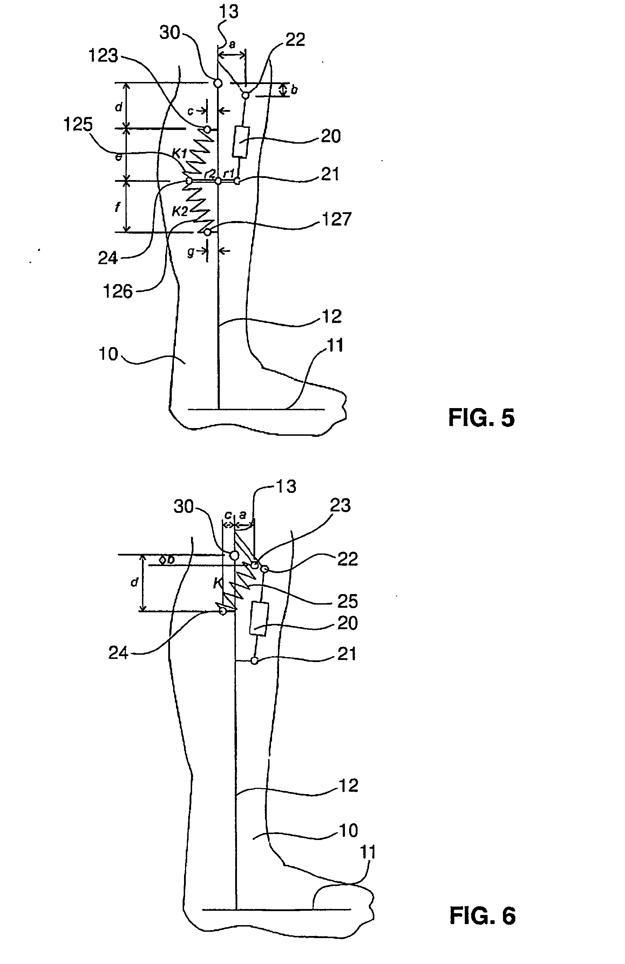

The artificial leg as an active prosthesis with an actuator can comprise a parallel spring as shown in Fig. 1 or 2, a serial spring assembly as shown in Fig, 5 or a combination of a parallel spring and a serial spring assembly with the elements shown in Fig. 1 and Fig. 5.

Further embodiments of the invention are laid down in the dependent claims.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,
- Fig. 1: shows a schematical representation of parts of an artificial leg according to an embodiment of the invention using a linear actuator and a parallel spring;
- Fig. 2: shows a schematical representation of parts of an artificial leg according to a further embodiment of the invention using a rotary actuator and a parallel spring;
- Fig. 3: shows a diagram of knee torque values against the knee flexion angle for an embodiment according to Fig. 1;
- Fig. 4: shows a diagram of knee torque values against the knee flexion angle for an embodiment according to Fig. 2;
- Fig. 5: shows a further embodiment according to further principle of the invention with an actuator and a serial spring;
- Fig. 6: shows the embodiment according to Fig. 1 reflecting specific design values for the distances relating to the attachment points; and
- Fig. 7A, 7B and 7C: show three different flexion positions of the artificial leg according to Fig. 1.

### DESCRIPTION OF PREFERRED EMBODIMENTS

Fig. 1 shows a schematical representation of parts of an artificial leg 10 according to an embodiment of the invention. The parts as shown are from the foot to the thigh. They comprise the inner skeleton structure of the artificial leg 10. The foot is shown with its foot longitudinal axis 11, oriented essentially horizontally. Perpendicular to the foot longitudinal axis 11 is provided the shank longitudinal axis 12 connecting the foot portion with the knee portion. The knee portion of the artificial leg is represented by the knee joint 30. Knee joint 30 comprises usually an axis being perpendicular to the crus longitudinal axis 12 as well as perpendicular to the foot longitudinal axis 11. Beyond the knee joint 30 and as a counter part to the shank portion is provided a thigh portion, represented by the thigh longitudinal axis 13. Said axis is perpendicular to the knee joint 30. In the representation of Fig. 1 the thigh longitudinal axis 13 is shown as prolongation of the shank longitudinal axis 12, i.e. the artificial leg is fully extended. Fig. 1 does not show the attachment portion or interface of the thigh longitudinal axis 13 with the remaining leg of the user nor does it show other components between the structural axes 11, 12, 13, movable parts as the joint 30 and any cover of the artificial leg 10. The artificial leg 10 can also only comprise the necessary technical elements, then the representation of the physiological elements of the leg in the figures only explain the layout.

In order to flex the artificial leg, an actuator 20 is provided being attached on one side at the thigh longitudinal axis 13 and on the other side at the shank longitudinal axis 12, i.e. on both sides of knee joint 30. Actuator 20 is a linear active actuator extending or reducing the distance between the attachment points 21 and 22. Lower attachment point 21 provides the connection with the shank longitudinal axis 12 through a web portion and upper attachment point 22 provides the connection with the thigh longitudinal axis 13 through a further web portion.

In the upright position of the leg as shown in Fig. 1, main axis of the actuator 20 is not parallel to the shank longitudinal axis 12, it can be seen that the lower attachment point 21 is nearer to the shank longitudinal axis 12 than to the upper attachment point 22. The upper attachment point 22 is lower than the knee joint 30 in the representation. When the actuator 20 is extended, then the artificial leg is flexed.

Additionally to actuator 20 providing an active element, a spring 25 is attached between lower attachment point 24 and higher attachment point 23. Spring 25 is in a position in Fig. 1 where it does only exert little torque.

The four attachment points 21, 22, 23 and 24 are preferably positioned in planes comprising the shank longitudinal axis 12, so that it can be stated that lower attachment point 24 is positioned opposite to lower attachment point 21. Upper attachment points 23 and 22 are positioned near one to another on the same side of the axis 12. It is possible that spring 25 and actuator 20 are attached to the same web connecting the points 23 and 22 to the thigh axis 13.

Spring 25 is provided in parallel to actuator 20, since the attachment points 21 and 24 provide connection to the shank structure and attachment points 22 and 23 provide connection to the thigh structure.

Fig. 2 shows a different embodiment of an artificial leg according to the invention. Same features receive the same reference numerals and similar features receive similar numerals. The embodiment of Fig. 2 uses a rotary actuator 130, i.e. an actuator 130 being attached at the knee joint 30 and acting directly on the thigh and shank longitudinal axes 12 and 13, rotating them one against the other. Here spring 25 is attached in the same way as in Fig. 1, providing a torque in parallel to the torque provided by the rotary actuator 130.

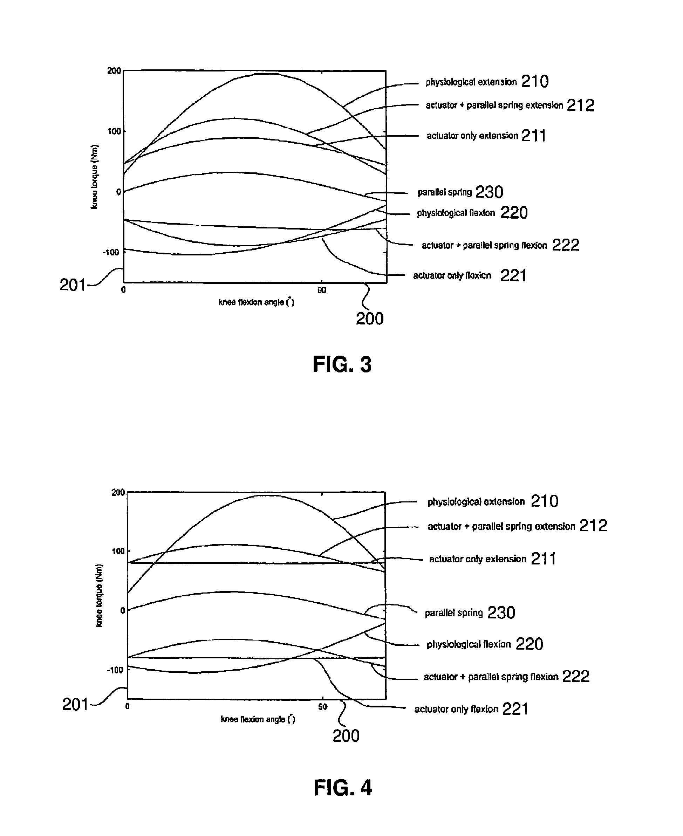

Fig. 3 shows a diagram of different knee torque values against the knee flexion angle for an embodiment according to Fig. 1. The knee torque 201 is shown for knee flexion angles for a value between 0 degree and approx. 120 to 130 degree. The knee flexion angle value of 0 degree is shown in the representation of the position of the axes 12 and 13 in Fig. 1. Several different torque values are shown in Fig. 3.

On a comparative basis the physiological extension 210 as well as the physiological flexion 220 is shown. The physiological extension 210 has a maximum value for an extension of just below 90 degree flexion angle, e.g. between 60 and 80 degree. The physiological flexion 220 has a minimum value for a flexion of just below 90 degree flexion angle, e.g. between 60 and 80 degree. The values for the physiological knee are based on D. E. Anderson, M. L. Madigan, M. A. Nussbaum, "Maximum voluntary joint torque as a function of joint angle and angular velocity: Model development and application to the lower limb", Journal of Biomechanics, vol. 40 (14), pp. 3105-3113, 2007.

The actuator 20 of the artificial leg 10 is now supposed to change the rotation angle of the knee joint 30, i.e. rotate the axes 12 and 13 one against the other. The torque exerted by the actuator 20 according to Fig. 1 is shown as curve 211 for the actuator-only-extension, providing a curve with a maximum but with lesser torque values at the maximum value angle. However, the absolute value depends on the employed actuator, here only an example is shown. Note, that in the present disclosure positive torques indicate extension torques, negative torques indicate flexion torques.

On the other side the torque exerted by the actuator 20 according to Fig. 1 is shown as curve 221 for the actuator-only-flexion, providing a curve with a minimum but with lesser torque values at the minimum value angle, exactly mirroring the extension torque, with the assumption that the linear actuator is equally strong in both directions. When a hydraulic or pneumatic cylinder is used, the flexion torque curve of the actuator will not be a mirror image of the extension torque, but the principles applied when using the spring 25 will remain valid. In this situation spring 25 is to be taken into account. The torque value exerted by spring 25 is shown as curve 230. This curve depends on the predetermined physical parameters of distances between joint 30 and the attachment points 23, 24 in view of the axes 12 and 13 as well as of course the actual spring stiffness values. Spring 25 is positioned in the embodiment shown in Fig. 1 in a way that at a flexion angle of 0 degree the spring does only exert little torque.

Spring 25 acts in any case additionally to the force of the actuator 30 for any flexion or extension movement. Therefore, the exerted torque has to be added and the resulting values are shown as curve 212 for actuator+spring-extension and as curve 222 for actuator+spring-flexion. It can clearly be seen that he parallel position of the spring 25 improves the spring extension curve 212 with a higher maximum torque, i.e. supports the action of actuator 20. On the other side the sum of actuator torque and spring torque add to curve 222 which has a lesser pronounced minimum value for the torque or even no minimum at all, which is acceptable because physiological flexion is weaker than extension.

Fig. 4 shows a diagram of knee torque values against the knee flexion angle for an embodiment according to Fig. 2. Therefore, Fig. 4 shows similar curves as Fig. 3 in relation to the rotary embodiment according to Fig. 2. Of course curves 210 and 220 for the physiological values are identical to the same curves of Fig. 3. The same is true for the torque values of the parallel spring 25, since it is positioned in a similar way. Since the rotary actuator 130 provides the same torque for every position of the axes 12 and 13, the actuator-only-extension curve 211 is a horizontal curve displaying a constant torque value over the whole range of angles. Therefore, the actuator+parallel-spring-extension curve 212 follows the curve of spring 25 with a smaller maximum value. On the other side the actuator 130 provides a constant negative torque value 221. This of course leads to the actuator+parallel-spring-flexion curve 222 which has the opposite direction compared to the physiological value curve. But since physiological flexion is weaker than extension, this is still acceptable.

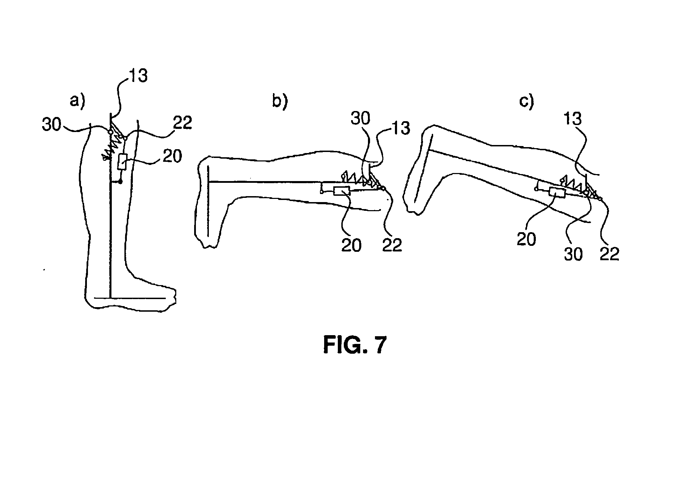

Fig. 7A, 7B and 7C show three different flexion positions of the artificial leg 10. Fig. 7A shows the same position of shank and thigh longitudinal axes along one single line, i.e. a flexion angle of 0 degree. Fig. 7B shows the shank longitudinal axis 12 in a horizontal position, whereas the thigh longitudinal axis 13 is still in the same orientation as in Fig. 7A, only positioned lower. Since the upper attachment point 22 did not change its rotary position, it is the spring 25 which has rotated around said attachment point 22 through turning around the knee joint 30, so that spring 25 is now oriented differently and stretched to a greater length. Fig. 7C finally shows a larger flexion of between 110 and 135 degree. Thigh longitudinal axis 13 of the artificial leg 10 is still oriented as in Fig. 7A and 7B. When the knee is more or less straightened only a small extension torque is created which would allow the artificial leg to go, without force provided by the actuator and neglecting friction and gravity, into the extension end stop. Fig. 7B shows the situation for a 90 degree flexion angle, where almost no torque is created, because the lever arm of the spring about the knee joint is very small. If the knee is flexed even more beyond 90 degree, a small flexion torque is created, which allows the user of the artificial leg that the knee would go, without force provided by the actuator and neglecting friction and gravity, into the flexion end stop. The use of the parallel spring allowing such a behaviour is based on principles shown in Fig. 6.

Fig. 6 shows the embodiment according to Fig. 1 reflecting specific design values for the distances relating to the attachment points as follows: One side of the parallel spring is attached on the thigh part in front of the knee, the other one is placed lower, on the shank part, behind the knee (when looking at the sagittal plane), and lateral to the actual knee joint. When the knee starts flexing from the fully extended position, the spring tension increases, and hence, it's force. When knee flexion reaches around 90 degrees, the spring and, hence, its line of action, lies exactly on the knee joint, such that no moment is exerted about the knee joint. The condition for zero torque at exactly 90 degrees flexion can be achieved with the variables indicated below, wherein the variables are mentioned in Fig. 6.

| Variable | value |
|---|---|
| a | 40.4 mm |
| b | 12.6 mm |
| c | 8.2 mm |
| d | 10.3 mm |
| K | 20 N/mm |

In view of control for the parallel spring, the torque generated by the parallel spring is a function of the knee angle. By measuring the knee angle, which is necessary in actuated prostheses to enable control of the knee angle in some way, the torque generated by the parallel spring can be determined.

Fig. 5 shows a further embodiment according to further principle of the invention with an actuator 20 attached with the upper attachment point 22 at the thigh longitudinal axis 13 as with the embodiment of Fig. 1 but the lower attachment point 21 is not attached directly connected via a web to the axis 12 but is hinged with a two-arm-lever hinged at the axis 12 where on the opposite side the lower attachment point 24 constitutes the other free end of the lever.

Here two springs 125 and 126 are provided to be able to act into both directions of the lever. There are provided in a serial relationship to the actuator 20, to allow accurate force control and protect the actuator 20 from shocks.

Either separated from the use of a parallel spring 25 according to Fig. 1 or in addition to this use, the serial springs according to Fig. 5 can be applied to the artificial leg 10. It is preferred to add such a serial elasticity to enable a better force control and to protect the actuator 20 against shocks. Fig. 5 shows the use of spiral springs. In order to provide pulling and pushing action, two pretensioned extension springs 125 and 126 are used. In order to reduce forces acting on the springs, a further transmission is used. This transmission comprises a rocker with two different lever arms. The rocker is attached at the shank axis 12 and the shorter lever arm r1 connects the attachment point 21 whereas the longer lever arm r2 connects the attachment point 24. Attachment point 24 is the lower attachment point 24 in view of the actuator but an intermediate attachment point for springs 125 and 126.

The deflection of the spring determines its force. The deflection of the series springs is given by the rotation (relative to the shank) of the rocker member that connects the linear actuator 20 and the springs 125 and 126. This rotation can easily be measured by a rotational sensor such as a potentiometer or an encoder. This signal can then be used for force control.

An example configuration of the series spring is given with the following values:

| Variable | value |
|---|---|
| a | 20 mm |
| b | 40 mm |
| c | 70 mm |
| d | 67 mm |
| e | 123 mm |
| f | 123 mm |
| g | 70 mm |
| r1 | 15 mm |
| r2 | 80 mm |
| K1 | 10 N/mm |
| K1 | 10 N/mm |

As the two springs 125 and 126 connected to the rocker member are in parallel to each other, the total stiffness is the sum of each individual stiffness. They do not necessarily need to be the same, but are in this example. The symmetry of the two springs 125 and 126 is also not a requirement. In this example they are extension springs, but other elastic elements could be used, as for example rubber bands.

### LIST OF REFERENCE SIGNS

- 10: artificial leg
- 11: foot longitudinal axis
- 12: shank longitudinal axis
- 13: thigh longitudinal axis
- 20: linear actuator
- 21: lower actuator attachment point
- 22: upper actuator attachment point
- 23: lower parallel spring attachment point
- 24: upper attachment point
- 25: parallel spring
- 30: rotation axis
- 123: upper serial spring attachment point
- 124: middle serial spring attachment point
- 125: upper serial spring
- 126: lower serial spring
- 127: lower serial spring attachment point
- 130: rotary actuator
- 200: knee flexion angle
- 201: knee torque
- 210: physiological extension
- 211: actuator only, extension
- 212: actuator + parallel spring, extension
- 220: physiological flexion
- 221: actuator only, flexion
- 222: actuator + parallel spring, flexion
- 230: parallel spring action

## Claims

1. Powered prosthesis with serial and/or parallel compliance, comprising a lower member (12) and an upper member (13), connected via a rotary joint (30; 130), further comprising an actuator (20; 130) connected with the lower member (12) and the upper member (13) to impart rotary motion to said lower member (12) and upper member (13) and at least one spring (25; 125, 126) connected with the actuator (20; 130), **characterized in that** the spring (25) is connected with actuator (20; 130) in parallel and is attached between the lower member (12) and the upper member (13) at predetermined attachment points (23, 24) adapted that the force exerted by the parallel spring (25) has a positive actuator supporting force value for a flexion angle between the lower member (12) and the upper member (13) between 20 and 90 degrees.

2. Prosthesis according to claim 1, wherein the torque exerted by the spring (25) is near 0 at a flexion angle of 0 degree between the two members (12, 13).

3. Prosthesis according to claim 1 or 2, wherein the torque exerted by the spring (25) is negative (flexion).

4. Prosthesis according to any one of claims 1 to 3, wherein the actuator (20; 130) is a linear actuator (20) and attached between the lower member (12) and the upper member (13) at attachment points (21, 22).

5. Prosthesis according to any one of claims 1 to 4, wherein the attachment point (22) of the actuator (20) at the upper member (13) is provided beyond or below (b) the axis line of the upper member (13).

6. Prosthesis according to any one of claims 1 to 3, wherein the actuator (20; 130) is a rotary actuator (130) and provided in the connection between the lower member (12) and the upper member (13).

7. Powered prosthesis with serial and/or parallel compliance according to the preamble of claim 1 and/or according to any of claims 1 to 5, **characterized in that** there are provided two springs (125, 126) connected with the actuator (20) in series, wherein is provided a rocker (r1, r2) attached at the lower member (12) providing two rocker attachment points (21, 24), wherein the actuator (20) is attached between the lower member (12) at one of the rocker attachment points (21) and the upper member (13) at a further attachment point (22), wherein the two springs (125, 126) are attached at the lower member (12) at predetermined attachment points (123, 127) and at the rocker (r2, r1) at the remaining rocker attachment (24).

8. Prosthesis according to claim 7, wherein the rocker arm (r1) connected with the actuator (20) is shorter than the rocker arm (r2) connected with the springs (125, 126).

9. Prosthesis according to claim 7 or 8, wherein the rocker attachment at the lower member (12) is offset (c, g) from the line between the attachment points (123, 127) of the springs (125, 126) with the lower member (12).

10. Prosthesis according to any one of claims 7 to 9, wherein the attachment point (22) of the actuator (20) at the upper member (13) is provided beyond or below (b) the axis line of the upper member (13).

11. Prosthesis according to any one of claims 1 to 10, wherein the prosthesis is a knee prosthesis for an artificial leg (10), wherein the lower member (12) is a shank replacement, the upper member (13) is a thigh replacement and the rotary joint (30; 130) is a knee joint.
